(19) Europäisches Patentamt
European Patent Office
Office européen des brevets

(11) **EP 3 498 147 B1**

(12) **EUROPÄISCHE PATENTSCHRIFT**

(45) Veröffentlichungstag und Bekanntmachung des Hinweises auf die Patenterteilung:
**30.07.2025 Patentblatt 2025/31**

(21) Anmeldenummer: **18212202.8**

(22) Anmeldetag: **13.12.2018**

(51) Internationale Patentklassifikation (IPC):
*A61B 1/00* (2006.01)    *H02N 2/10* (2006.01)
*H02N 2/04* (2006.01)    *A61B 34/00* (2016.01)
*A61B 17/00* (2006.01)

(52) Gemeinsame Patentklassifikation (CPC):
**A61B 1/00133; A61B 34/71; H02N 2/046;**
A61B 1/00087; A61B 2017/00402

(54) **LÄNGLICHES SCHAFTINSTRUMENT**

ELONGATED SHAFT INSTRUMENT

INSTRUMENT OBLONG À TIGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.12.2017 DE 102017222866**

(43) Veröffentlichungstag der Anmeldung:
**19.06.2019 Patentblatt 2019/25**

(73) Patentinhaber: **Richard Wolf GmbH**
**75438 Knittlingen (DE)**

(72) Erfinder: **TEICHTMANN, Elmar**
**75015 Bretten (DE)**

(74) Vertreter: **Patentanwälte Hemmer Lindfeld Frese Partnerschaft mbB**
**Wallstraße 33a**
**23560 Lübeck (DE)**

(56) Entgegenhaltungen:
EP-A2- 1 098 429        WO-A1-2014/005949
WO-A1-2014/142242        DE-A1- 102005 024 317
DE-A1- 102006 027 385        DE-A1- 102006 027 409
US-A- 4 888 515

**Beschreibung**

Die vorliegende Offenbarung betrifft ein längliches Schaftinstrument, wie beispielsweise ein minimalinvasives medizinisches Instrument, ein Endoskop oder Technoskop, an dem sich distalseitig ein bewegliches Bauteil und/oder betätigbarer Instrumentenkopf befindet. Insbesondere betrifft die Offenbarung Instrumente, bei denen der Instrumentenkopf gegenüber der Längsrichtung gesteuert, insbesondere robotergesteuert, mechanisch abwinkelbar ist.

Minimalinvasive medizinische Instrumente mit antreibbarem und/oder steuerbarem Instrumentenkopf am distalen Ende sind bereits bekannt. Beispielsweise beschreibt die DE 10 2015 215 469 A1 ein medizinisch-endoskopisches Instrument, mit dem über Seilzüge eine Zange am distalen Ende betätigt wird.

Ein robotersteuerbares Endoskop ist beispielsweise aus der US 7,297,142 B2 bekannt. Dort wird ein Instrumentenkopf ebenfalls über eine Mehrzahl von Zugseilen abgeknickt und betätigt. Die Robotersteuerung über Zugseile ist allerdings technisch recht aufwändig.

Die DE 10 2006 027385 A1 beschreibt eine Festkörperaktor-Antriebsvorrichtung mit einem piezoelektrischen Antrieb. Die EP 1 098 429 A2 beschreibt einen ähnlichen elektromechanischen Motor.

Das hierin offenbarte längliche Schaftinstrument erlaubt dagegen eine genaue Steuerung und/oder Betätigung eines distalseitigen Bauteils oder Instrumentenkopfs auf einfachere Weise. Zudem ist zum Beispiel ein Abknickwinkel eines distalseitigen Bauteils oder Instrumentenkopfs nicht begrenzt. Bei Bedarf kann das Instrument zur endlosen Drehung eines distalseitigen Bauteils oder Instrumentenkopfs verwendet werden.

Das Schaftinstrument gemäß der vorliegenden Offenbarung hat gegenüber den bekannten Antrieben den Vorteil, dass keine Seilzüge benötigt werden, um ein distalseitiges Bauteil oder einen Instrumentenkopf am distalen Ende des Schafts zu bewegen und/oder zu betätigen.

Gemäß einem ersten Aspekt der vorliegenden Offenbarung wird ein längliches Schaftinstrument mit einem Antriebssystem bereitgestellt mit zwei sich im Wesentlichen parallel zueinander in einer Längsrichtung oszillatorisch beweglichen Impulsgebern, einem um eine Drehachse drehbar gelagerten Abtriebsrad zum Antreiben einer Bewegung und/oder Betätigung eines distalseitigen Bauteils oder Instrumentenkopfs des Schaftinstruments, und einem translatorisch begrenzt beweglichen Antriebsrad zum Kraft-, Form- und/oder Reibschluss mit dem Abtriebsrad, wobei das Antriebsrad durch die zwei Impulsgeber antreibbar in zwei Oszillationsrichtungen oszillatorisch beweglich ist.

Eine oszillatorische Bewegung der Impulsgeber ist besonders einfach für ein roboter- bzw. computergesteuertes System zu realisieren. Die Impulsgeber können beispielsweise oszillatorisch anregbare piezoelektrische Aktoren am distalen Schaftende und/oder Schub-/Zugmittel als Betätigungselemente aufweisen, die beispielweise jeweils proximalseitig von piezoelektrischen Aktoren in eine oszillatorisch axiale Bewegung anregbar sind und diese oszillatorisch axiale Bewegung auf das Antriebsrad übertragen. Unter "oszillatorischer Bewegung" oder "Oszillation" sei hier eine irgendwie ausgeartete einmalige oder mehrmalige Hin- und Herbewegung gemeint, d.h. es kann eine sinusförmige Schwingung sein oder gepulst, rechteckig, sägezahnförmig, kontinuierlich, diskontinuierlich, periodisch oder aperiodisch. Das Abtriebsrad kann durch die radiale Außenfläche einer Abtriebswelle gebildet sein, die mit dem distalseitigen Bauteil oder Instrumentenkopf zu dessen Bewegung und/oder Betätigung gekoppelt ist. Das distalseitige Bauteil oder der distalseitige Instrumentenkopf kann beispielsweise ein gegenüber einer Längsrichtung des Schaftinstruments abwinkelbares Skalpell, eine Schere, eine Zange, eine Kamera, eine Leuchte oder eine beliebige Kombination aus diesen sein. Es können in einem Schaftinstrument zwei oder mehr Antriebssysteme dieser Art integriert sein, um beispielsweise mehrere Bewegungsfreiheitsgrade des distalseitigen Bauteils oder Instrumentenkopfs oder eine Mehrzahl von Bauteilen unabhängig voneinander antreiben zu können.

Optional kann das Abtriebsrad durch einen abwälzenden Kraft-, Form- und/oder Reibschluss zwischen einer radialen Fläche des Antriebsrads und einer radialen Fläche des Abtriebsrads vom Antriebsrad kontinuierlich antreibbar sein. Der sich abwälzende Kraft-, Form- und/oder Reibschluss zwischen der radialen Fläche des Antriebsrads und der radialen Fläche des Abtriebsrads erlaubt eine Umsetzung der Oszillationsbewegung der Impulsgeber in eine kontinuierliche Drehung des Abtriebsrads. Eine stufenlose gleichmäßige Drehung des Abtriebsrads ist also dadurch möglich, wobei die Drehgeschwindigkeit über die Oszillationsfrequenz der Impulsgeber bestimmt ist.

Optional können die zwei Oszillationsrichtungen zueinander im Wesentlichen orthogonal verlaufen. Damit wird auf einfache und platzsparende Weise eine Oszillationsebene aufgespannt, die vorzugsweise im Wesentlichen orthogonal zur Drehachse liegt.

In einer bevorzugten Ausführungsvariante kann das Antriebsrad beispielsweise als ein das Abtriebsrad exzentrisch umgreifendes Hohlrad ausgeführt sein. Die radiale Fläche des Antriebsrads kann dann eine radiale Innenfläche und die radiale Fläche des Abtriebsrads kann dann eine radiale Außenfläche sein. Der Radius der Innenfläche des Antriebsrads ist dann größer als der Radius der radialen Außenfläche des Abtriebsrads. Die Differenz zwischen dem Radius der Innenfläche des Antriebsrads und dem Radius der radialen Außenfläche des Abtriebsrads kann im Wesentlichen einem exzentrischen Versatz zwischen dem Antriebsrad und dem Abtriebsrad entsprechen. Damit wird ein ausreichender Bewegungsspielraum zum Abwälzen eines Kraft-, Form- und/oder Reibschlusses zwischen dem Abtriebsrad und dem Antriebsrad bereitgestellt.

In einer dazu alternativen, hierin aber nicht näher beschriebenen Ausführungsvariante kann das Abtriebsrad als Hohlrad ausgeführt sein, wobei das Antriebsrad exzentrisch in das Abtriebsrad eingreift. Die radiale Fläche des Antriebsrads kann dann eine radiale Außenfläche und die radiale Fläche des Abtriebsrads eine radiale Innenfläche sein. Das translatorisch und rotatorisch beschränkt bewegliche Antriebsrad kann bei dieser Ausführungsvariante als Stirnrad ausgeführt sein und seitlich in die Hohlradkontur des Abtriebsrad eingreifen, um dieses von innen anzutreiben.

Optional können das Antriebsrad und das Abtriebsrad miteinander korrespondierende Verzahnungen aufweisen, wobei ein abwälzender Kraft-, Form- und/oder Reibschluss durch einen teilumfänglichen und umlaufenden Eingriff der Verzahnungen bewirkt wird. Dies ist besonders vorteilhaft, um Schlupf zu vermeiden. Alternativ oder zusätzlich können die radiale Fläche des Antriebsrads und die radiale Fläche des Abtriebsrads miteinander korrespondierende Reibflächen aufweisen.

Optional können die Verzahnungen zykloidisch sein und das gleiche Modul aufweisen, wobei die Zähnezahl auf dem Abtriebsrad und die Zähnezahl auf dem Antriebsrad ein Untersetzungsverhältnis zwischen der Oszillationsfrequenz der Impulsgeber und der Drehfrequenz des Abtriebsrads bestimmen. Bei miteinander korrespondierenden Reibflächen können der Radius der radialen Fläche des Abtriebsrads und der Radius der radialen Fläche des Antriebsrads das Untersetzungsverhältnis bestimmen. Bei hohem Untersetzungsverhältnis bedarf es vieler Oszillationen der Impulsgeber für eine ganze Drehung des Abtriebsrads bzw. eine Oszillation der Impulsgeber treibt das Abtriebsrad nur wenig weiter. Dies kann gewünscht sein, um eine sehr genaue Steuerung der Bewegung und/oder Betätigung des distalseitigen Bauteils oder Instrumentenkopfs zu erzielen. Die Oszillationsfrequenz der Impulsgeber kann entsprechend hoch eingestellt werden, um eine schnelle Drehung des Abtriebsrads zu erzielen. Beispielsweise kann das Abtriebsrad die Zähnezahl $Z_1$=50 und das Antriebsrad die Zähnezahl $Z_2$=51 aufweisen, sodass sich ein Untersetzungsverhältnis $Z_1/(Z_2-Z_1)$ von 50:1 ergibt, d.h. es werden 50 Oszillationen der Impulsgeber benötigt, um das Abtriebsrad um 360° zu drehen. Mit einer Oszillationsfrequenz der Impulsgeber von 10 Hz lässt sich dann beispielsweise eine Winkelgeschwindigkeit von 72° pro Sekunde erzielen.

Optional kann die Zahnhöhe der Verzahnungen kleiner sein als ein exzentrischer Versatz zwischen dem Antriebsrad und dem Abtriebsrad. Damit wird ein ausreichender Bewegungsspielraum zum Abwälzen eines Kraft-, Form- und/oder Reibschlusses zwischen Abtriebsrad und Antriebsrad bereitgestellt. Für einen möglichst platzsparenden Aufbau kann die Zahnhöhe der Verzahnungen allerdings nur minimal kleiner sein als ein exzentrische Versatz zwischen Abtriebsrad und Antriebsrad und beispielsweise 50% bis 99%, vorzugsweise 90% bis 99%, des exzentrischen Versatzes betragen.

Optional können die Impulsgeber dazu ausgestaltet sein, das Antriebsrad im Wesentlichen 90° phasenverschoben zueinander oszillatorisch anzutreiben, sodass es einer Kreisbahn im Wesentlichen parallelverschoben folgt, wobei der Radius der Kreisbahn im Wesentlichen einem exzentrischen Versatz zwischen dem Antriebsrad und dem Abtriebsrad entspricht. Mit "parallelverschoben" sei hier eine rein translatorische Verschiebung gemeint, der im Wesentlichen keine rotatorische Bewegung überlagert ist. "Im Wesentlichen" soll in diesem Zusammenhang bedeuten, dass dabei geringfügige rotatorische Kippbewegungen im Rahmen von ±5° auftreten können. Vorzugsweise folgt das Antriebsrad allerdings exakt parallelverschoben einer Kreisbahn, also ohne jegliche überlagerte rotatorische Bewegung.

Optional kann die Drehrichtung des Abtriebsrads durch eine Phasenverschiebung zwischen den Impulsgebern bestimmt sein. Insbesondere das Vorzeichen der Phasenverschiebung kann hier bestimmend sein. Beispielsweise kann eine Phasenverschiebung um +90° eine Drehung des Abtriebsrads im Uhrzeigersinn antreiben und eine Phasenverschiebung um -90° eine Drehung des Abtriebsrads gegen den Uhrzeigersinn. Entsprechend ist die Bewegung- bzw. Betätigungsrichtung des distalseitigen Bauteils oder Instrumentenkopfs über die Phasenverschiebung steuerbar.

Optional kann das Abtriebsrad zum Abwinkeln des distalseitigen Bauteils oder Instrumentenkopfs gegenüber der Längsrichtung ausgestaltet sein, wobei die Drehachse im Wesentlichen orthogonal zur Längsrichtung verläuft. Alternativ dazu kann die Drehachse beispielsweise parallel zur Längsrichtung verlaufen und eine Drehbewegung des distalseitigen Bauteils oder Instrumentenkopfs um die Längsrichtung bewirken.

Die Impulsgeber weisen jeweils ein längliches sich in Längsrichtung erstreckendes Betätigungselement auf. Solch ein Betätigungselement kann beispielsweise eine Schub-/Zugstange oder ein hydraulischer Fluidkanal sein. Dadurch muss ein piezoelektrischer Aktor nicht innerhalb des sehr begrenzten Bauraums am distalen Ende eines Schaftinstruments angeordnet werden, sondern kann proximalseitig vom Schaft des Schaftinstruments das Betätigungselement zu axialen Schwingungen anregen, die es über die Länge des Schafts auf das Antriebsrad überträgt.

Das Antriebsrad ist durch einen ersten der zwei Impulsgeber antreibbar in eine erste der Oszillationsrichtungen oszillatorisch beweglich. Das Antriebsrad ist dabei durch einen zweiten der zwei Impulsgeber antreibbar in eine zweite der Oszillationsrichtungen oszillatorisch beweglich. Dies ist für die Ansteuerung der Impulsgeber vorteilhaft, da sie unabhängig voneinander angetrieben werden können.

Alternativ oder zusätzlich dazu kann das Antriebsrad in eine erste der Oszillationsrichtungen oszillatorisch beweglich sein, wenn die Bewegung der Impulsgeber in der Summe ungleich null ist, und in eine zweite der Oszillationsrichtungen oszillatorisch beweglich sein, wenn sich die Impulsgeber relativ zueinander bewegen. Hierbei können die Impulsgeber zwar mechanisch gekoppelt sein, was eine höhere Herausforderung an die Ansteuerung stellt, aber einen einfacheren symmetrischen Aufbau mit geringerer Bauteilevielfalt ermöglicht.

Optional können die Impulsgeber jeweils distalseitig über ein Gelenksystem mechanisch an das Antriebsrad gekoppelt sein, wobei das Gelenksystem eine im Wesentlichen in Längsrichtung oszillierende Bewegung eines ersten der zwei Impulsgeber um einen ersten Winkel in eine erste der Oszillationsrichtungen des Antriebsrads umlenkt. Dieser erste Winkel kann durch das Gelenksystem fest vorgegeben sein oder von der relativen Position bzw. Bewegungsrichtung der Impulsgeber abhängen.

Optional kann das Gelenksystem eine im Wesentlichen in Längsrichtung oszillierende Bewegung eines zweiten der zwei Impulsgeber in eine zweite der Oszillationsrichtungen des Antriebsrads übertragen. Die zweite der Oszillationsrichtungen kann durch das Gelenksystem festgelegt in Längsrichtung verlaufen, sodass es keiner Umlenkung um einen zweiten Winkel bedarf. Alternativ oder zusätzlich dazu kann das Gelenksystem allerdings eine im Wesentlichen in Längsrichtung oszillierende Bewegung eines zweiten der zwei Impulsgeber um einen zweiten Winkel in eine zweite der Oszillationsrichtung des Antriebsrads umlenken, wobei die Summe des ersten und zweiten Winkels im Wesentlichen 90° beträgt. Auch dieser zweite Winkel kann durch das Gelenksystem fest vorgegeben sein oder von der relativen Position bzw. Bewegungsrichtung der Impulsgeber abhängen. Optional kann das Gelenksystem den ersten Winkel und den zweiten Winkel auf im Wesentlichen 45° festlegen.

Optional können die Impulsgeber jeweils distalseitig über mindestens einen Hebel mechanisch an das Antriebsrad gekoppelt sein, wobei der jeweils mindestens eine Hebel mit einer beweglichen Lagerachse am zugehörigen Impulsgeber und mit einer beweglichen Lagerachse am Antriebsrad angelenkt ist. Mit mindestens einem Hebel pro Impulsgeber ergeben sich in der Summe mindestens zwei Hebel insgesamt. Die Hebel können baugleich und symmetrisch bezüglich einer von der Längsrichtung und der Drehachse aufgespannten Spiegelebene angeordnet sein. Alternativ können die Hebel unterschiedlich geformt und nicht symmetrisch angeordnet sein.

Erfindungsgemäß sind die Impulsgeber über zwei Kopplungshebel mechanisch miteinander gekoppelt. Die Kopplungshebel können optional an mindestens einer zur Drehachse parallelen und bezüglich des Schafts ortsfesten Lagerachse drehbar gelagert sein.

Erfindungsgemäß sind die zwei Kopplungshebel derart miteinander verzahnt, dass die Lagerachsen immer symmetrisch zur Längsrichtung angeordnet sind. Dadurch wird eine überlagerte rotatorische Bewegung des Antriebsrads ausgeschlossen, sodass es exakt parallelverschoben wird.

Optional können das Antriebsrad und ein die Impulsgeber mit dem Antriebsrad koppelndes Gelenksystem symmetrisch bezüglich einer von der Längsrichtung und der Drehachse aufgespannten Spiegelebene aufgebaut sein. Die reduziert die Bauteilevielfalt und vereinfacht den Produktionsprozess.

Optional kann ein die Impulsgeber mit dem Antriebsrad koppelndes Gelenksystem, insbesondere mindestens ein Kopplungshebel, an mindestens einer zur Drehachse parallelen und bezüglich des Schafts ortsfesten Lagerachse drehbar gelagert sein.

Optional kann ein die Impulsgeber mit dem Antriebsrad koppelndes Gelenksystem, insbesondere zwei Kopplungshebel, an zwei zur Drehachse parallelen und bezüglich des Schafts ortsfesten Lagerachsen drehbar gelagert sein, wobei die Lagerachsen symmetrisch bezüglich einer von der Längsrichtung und der Drehachse aufgespannten Spiegelebene angeordnet sind. Auch hier reduziert der symmetrische Aufbau die Komplexität des Produktionsprozesses.

Optional kann das längliche Schaftinstrument, beispielsweise ein minimalinvasives medizinisches Instrument, ein Endoskop oder Technoskop, einen sich in einer Längsrichtung erstreckenden Schaft und ein distalseitig am Schaft befindliches bewegliches und/oder betätigbares Bauteil oder einen Instrumentenkopf aufweisen, wobei das Antriebssystem proximal vom Bauteil oder Instrumentenkopf angeordnet ist und die Impulsgeber in Längsrichtung durch den Schaft geführt sind.

Die Offenbarung ist nachfolgend anhand von in den Zeichnungen dargestellten Ausführungsbeispielen näher erläutert. Es zeigen:

Fig. 1 eine perspektivische Ansicht auf einen distalen Teil einer beispielhaften Ausführungsform des hierin offenbarten Instruments, die nicht Teil der Erfindung ist;

Fig. 2a-d Prinzipiendarstellungen von Funktionsweisen eines Antriebs für den Instrumentenkopf einer beispielhaften Ausführungsform des hierin offenbarten Instruments, die nicht Teil der Erfindung ist;

Fig. 3a-c Prinzipiendarstellungen zur Parallelverschiebung des Antriebsrads und der Phasenverschiebung einer beispielhaften Ausführungsform des hierin offenbarten Instruments, die nicht Teil der Erfindung ist;

Fig. 5 eine Seitenansicht auf das Abtriebsrad, das Antriebsrad, die Impulsgeber und das Gelenksystem einer ersten beispielhaften Ausführungsform des hierin offenbarten Instruments, die nicht Teil der Erfindung ist;

Fig. 6 eine Seitenansicht auf das Abtriebsrad, das Antriebsrad, die Impulsgeber und das Gelenksystem einer zweiten beispielhaften Ausführungsform des hierin offenbarten Instruments, die nicht Teil der Erfindung ist;

Fig. 7 zwei Seitenansichten auf das Abtriebsrad, das Antriebsrad, die Impulsgeber und das Gelenksystem einer erfindungsgemäßen dritten beispielhaften Ausführungsform des hierin offenbarten Instruments;

Fig. 8 zwei Seitenansichten auf das Abtriebsrad, das Antriebsrad, die Impulsgeber und das Gelenksystem einer vierten beispielhaften Ausführungsform des hierin offenbarten Instruments, die nicht Teil der Erfindung ist;

Fig. 9 zwei Seitenansichten auf das Abtriebsrad, das Antriebsrad, die Impulsgeber und das Gelenksystem einer fünften beispielhaften Ausführungsform des hierin offenbarten Instruments, die nicht Teil der Erfindung ist; und

Fig. 10 zwei Seitenansichten auf das Abtriebsrad, das Antriebsrad, die Impulsgeber und das Gelenksystem einer sechsten beispielhaften Ausführungsform des hierin offenbarten Instruments, die nicht Teil der Erfindung ist.

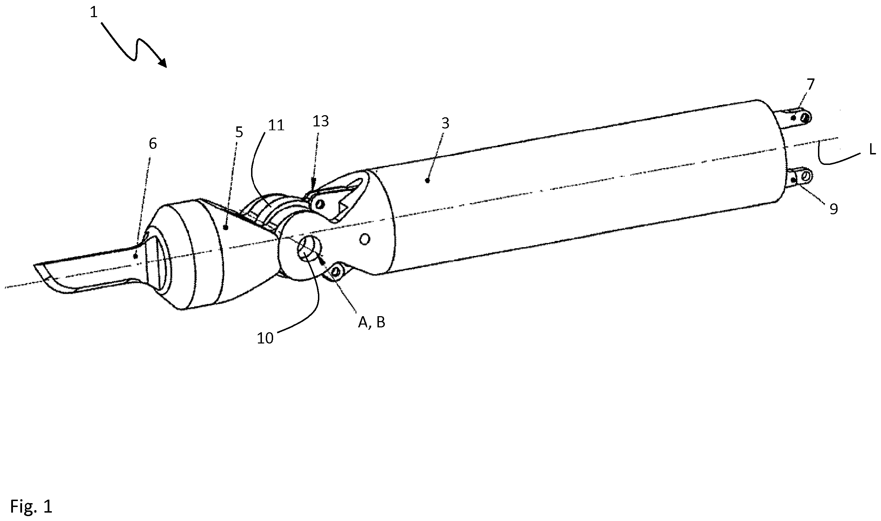

In Fig. 1 ist ein Schaftinstrument 1 in Form eines minimalinvasiven medizinischen Instruments mit einem sich in einer Längsrichtung L erstreckenden Schaft 3 gezeigt. Am distalen Ende des Schafts 3 befindet sich ein gegenüber der Längsrichtung L abwinkelbarer Instrumentenkopf 5 mit einer Skalpellklinge 6 am distalen Ende. Der Instrumentenkopf 5 ist dabei beweglich, indem er um eine senkrecht zur Längsrichtung L verlaufende Schwenkachse A durch ein im Schaftinstrument 1 integriertes Antriebssystem abwinkelbar ist. Durch das Innere des Schafts 3 erstrecken sich längliche Betätigungselemente 7, 9, die als Bestandteil oszillatorisch beweglicher Impulsgeber des Antriebssystems fungieren. Die Betätigungselemente 7, 9 sind in Form von Zug-/Schubstangen ausgebildet und mit einem Abstand zur Längsachse L des Schafts 3 diametral gegenüberliegend an lateralen Seiten des Schafts 3 angeordnet. Die Betätigungselemente 7, 9 können in axialer Richtung entlang der Längsrichtung L schwingen und somit als Bestandteil oszillatorisch beweglicher Impulsgeber fungieren. Die Schwenkachse A stimmt hier mit einer Drehachse B überein, um die ein Abtriebsrad 10 zum Antreiben der Abwinklung des Instrumentenkopfs 5 drehbar gelagert ist. Das Abtriebsrad 10 ist hier fest mit dem Instrumentenkopf 5 gekoppelt. Ein Antriebsrad 11 ist hier in Form eines Hohlrads ausgestaltet und umgreift das Abtriebsrad 10 exzentrisch bezüglich der Drehachse B bzw. der Schwenkachse A. Das Antriebsrad 11 ist translatorisch beschränkt beweglich und über ein Gelenksystem 13 mit den Betätigungselementen 7, 9 derart gekoppelt, dass es in zwei Oszillationsrichtungen oszillatorisch beweglich ist, wobei die Oszillationsrichtungen in einer Ebene senkrecht zur Drehachse B bzw. zur Schwenkachse A liegen. Die Oszillationen des Antriebsrads 11 in den zwei Oszillationsrichtungen sind also durch die zwei Betätigungselemente 7, 9 antreibbar. Das Abtriebsrad 10 ist durch einen abwälzenden Kraft-, Form- und/oder Reibschluss zwischen einer radialen Innenfläche des Antriebsrads 11 und einer radialen Außenfläche des Abtriebsrads vom Antriebsrad 11 kontinuierlich antreibbar.

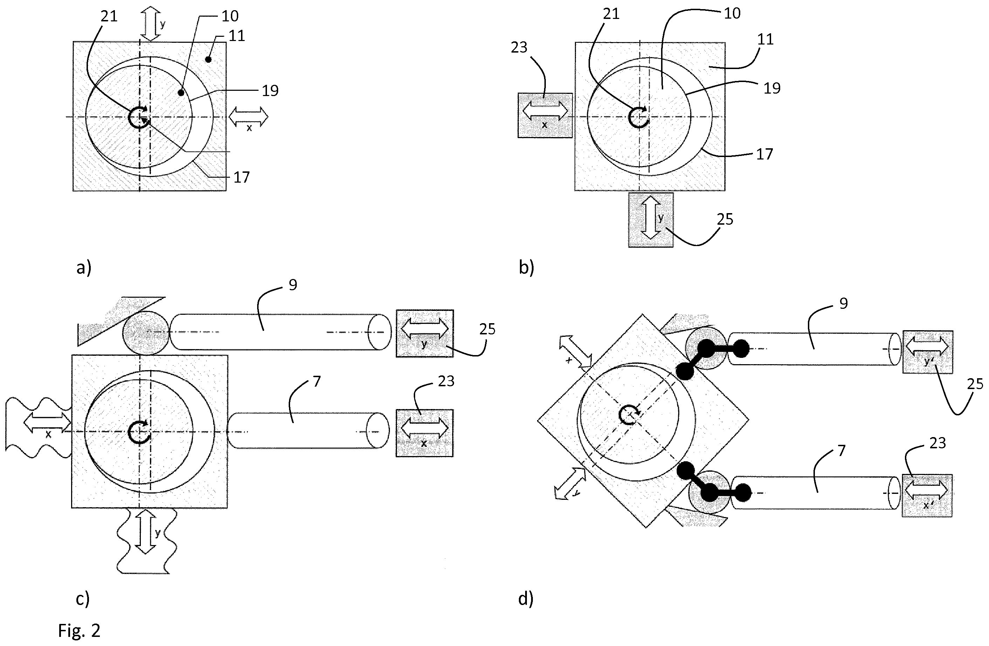

In Fig. 2 ist das Antriebsprinzip zwischen dem Antriebsrad 11 und dem Abtriebsrad 10 gezeigt. Fig. 2a zeigt wie das Antriebsrad 11 in eine erste Oszillationsrichtung x und eine dazu zweite Oszillationsrichtung y schwingen kann. Das Antriebsrad 11 weist eine im Wesentlichen zylindrische Innenfläche 17 mit einem Durchmesser $R_i$ auf. Das Antriebsrad 11 umgreift exzentrisch das Abtriebsrad 10, das eine im Wesentlichen zylindrische Außenfläche 19 mit einem Radius $R_a$ aufweist. Der Durchmesser $R_i$ der Innenfläche des Antriebsrads 11 ist größer als der Durchmesser $R_a$ der radialen Außenfläche 19 des Abtriebsrads 10. Das Abtriebsrad 10 ist ortsfest bezüglich des Schafts 3 drehbar um die Drehachse B gelagert und kann nicht in x- oder y-Richtung schwingen. Der exzentrische Versatz zwischen dem Antriebsrad 11 und dem Abtriebsrad 10 entspricht hier genau der Differenz $R_i$ - $R_a$. Damit ergibt sich in der in Fig. 2a gezeigten Position auf der linken Seite ein teilumfänglicher Kontakt und somit ein Reibschluss zwischen der radialen Innenfläche 17 des Antriebsrads 11 und der radialen Außenfläche 19 des Abtriebsrads 10. Schwingt das Antriebsrad 11 nun in den zwei Oszillationsrichtungen x und y mit einem relativen Phasenunterschied von 90° zueinander, so kann es einer Kreisbahn 21 im Wesentlichen parallelverschoben folgen. Beispielsweise kann die Schwingung in x-Richtung durch $x(t) = (R_i - R_a)\cdot\sin(2\pi ft)$ und die Schwingung in y-Richtung durch $y(t) = (R_i - R_a)\cdot\cos(2\pi ft)$ beschrieben sein, wobei f die Oszillationsfrequenz in Hz ist. Der Radius der sich ergebenden Kreisbahn 21 entspricht im Wesentlichen dem exzentrischen Versatz $R_i$ - $R_a$. Folgt das Antriebsrad 11 dieser Kreisbahn 21, so wälzt sich der teilumfängliche Reibschluss umlaufend mit der Oszillationsfrequenz f auf der radialen Außenfläche 19 des Abtriebsrads 10 ab. Dadurch wird ein Drehmoment auf das Abtriebsrad 10 übertragen. Anstatt eines Reibschlusses zwischen im Wesentlichen zylindrischen Flächen können die Innenfläche 17 des Antriebsrads 11 und die radiale Außenfläche 19 des Abtriebsrads 10 miteinander korrespondierende Verzahnungen aufweisen, um zusätzlich einen Kraft- bzw. Formschluss durch einen teilumfänglichen und umlaufenden Eingriff der Verzahnungen zu erzielen.

In Fig. 2b ist gezeigt wie die Oszillation des Antriebsrads 11 in x- und y-Richtung durch die entsprechend oszillatorisch beweglichen Impulsgeber 23, 25 antreibbar ist. Hier sind die Impulsgeber 23, 25 orthogonal zueinander angeordnet. In Fig. 2c ist eine alternative Antriebsmöglichkeit über zwei parallel zueinander angeordnete Impulsgeber gezeigt. Der Impulsgeber 23 übt hier Druckstöße in negative x-Richtung auf das Antriebsrad 11 aus, das auf der gegenüberliegenden Seite eine entsprechende Druckfeder oder Elastomerelement staucht, die das Antriebsrad 11 in positive x-Richtung zurückstellt. Die ebenfalls in positive x-Richtung ausgeübten Druckstöße des Impulsgebers 25 werden hier über eine Umlenkrolle um 90° in negative y-Richtung umgelenkt, sodass das Antriebsrad 11 in negative y-Richtung eine gegenüberliegende Druckfeder oder Elastomerelement staucht, die das Antriebsrad 11 in positive y-Richtung wieder zurückstellt. In Fig. 2d ist der Aufbau symmetrischer. Hier werden bei Oszillationen die Impulsgeber 23, 25 jeweils um jeweils 45° über eine jeweilige Umlenkrolle umgelenkt. Die sich ergebenden Oszillationsrichtungen x und y sind zueinander orthogonal, jedoch gegenüber den zuvor beschriebenen Oszillationsrichtungen um 45° gekippt.

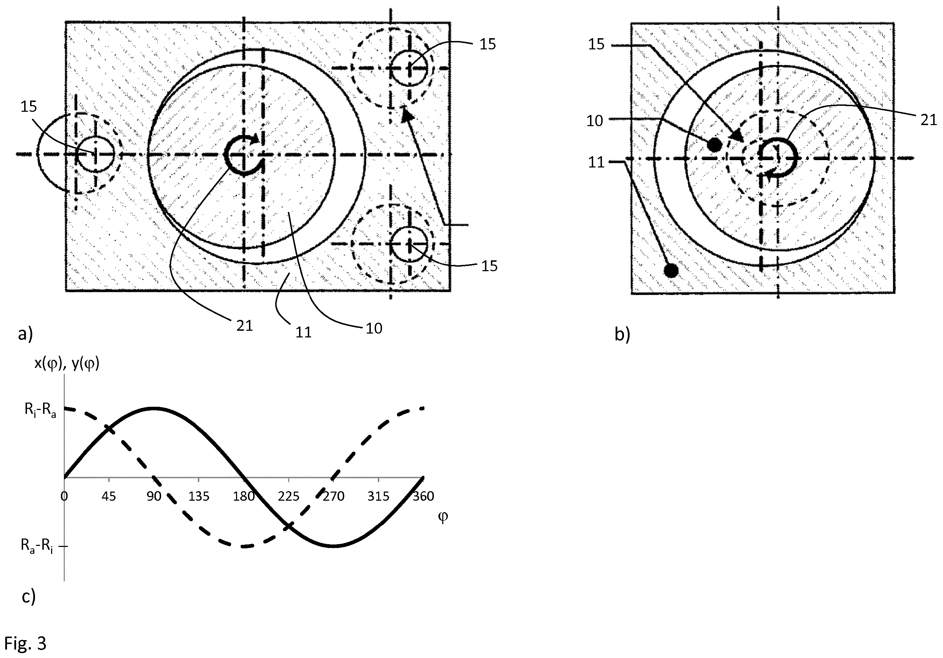

Fig. 3a und 3b zeigen zwei prinzipielle Möglichkeiten, eine möglichst exakte Parallelverschiebung des Antriebsrads 11 entlang einer Kreisbahn 21 zu erzwingen und somit überlagerte Rotationsbewegungen des Antriebsrads 11 zu vermeiden. Fig. 3a zeigt dazu eine Mehrzahl von Exzenterlagerungen 15 (hier drei), die eine rotatorische Bewegung des Antriebsrads 11 verhindern bzw. stark einschränken. In Fig. 3b ist eine zentrale Exzenterlagerung 15 dargestellt, welche die Einhaltung der Exzentrizität des Antriebsrads erzwingt, eine rotatorische Überlagerung aber nicht verhindern kann. Nachteilig an einer solchen Zwangsführung ist, dass der Kraft-, Form- und/oder Reibschluss zwischen Antriebsrad 11 und Abtriebsrad 10 jederzeit besteht und nicht voneinander gelöst werden kann. Im Fehlerfall ist es jedoch von Vorteil, wenn der Kraft-, Form- und/oder Reibschluss gelöst werden kann, um eine einfache Streckung des Instrumentenkopfgelenks und die Entnahme des Instruments durch einen Trokar aus dem Körper eines Patienten zu ermöglichen. In Fig. 3c ist der Phasenunterschied zwischen den Schwingungen x- und y-Richtung verdeutlicht. Die Amplitude der Schwingung entspricht hier dem exzentrischen Versatz $R_i - R_a$. Die Schwingungen sind hier als Funktion des Umlaufwinkels $\varphi(t)$ =$2\pi f\cdot t$ gezeigt, mit dem der teilumfängliche Kraft-, Form- und/oder Reibschluss auf der radialen Außenfläche 19 des Abtriebsrads 10 abwälzend umläuft. Die durchgezogene Linie stellt die Auslenkung in x-Richtung mit

$$x(\varphi) = (R_i - R_a) \cdot \sin\left(\frac{2\pi}{360°}\varphi\right)$$

und die gestrichelte Linie stellt die Auslenkung in y-Richtung mit $y(\varphi) = (R_i -$

$R_a) \cdot \cos\left(\frac{2\pi}{360°}\varphi\right)$ dar. In Fig. 3a ist eine Stellung des Antriebsrads 11 bei $\varphi$ = 90° mit x = $R_i - R_a$ und y = 0 gezeigt. In Fig. 3b ist das Antriebsrad 11 bei $\varphi$ = 270° mit x = $R_a - R_i$ und y = 0 gezeigt.

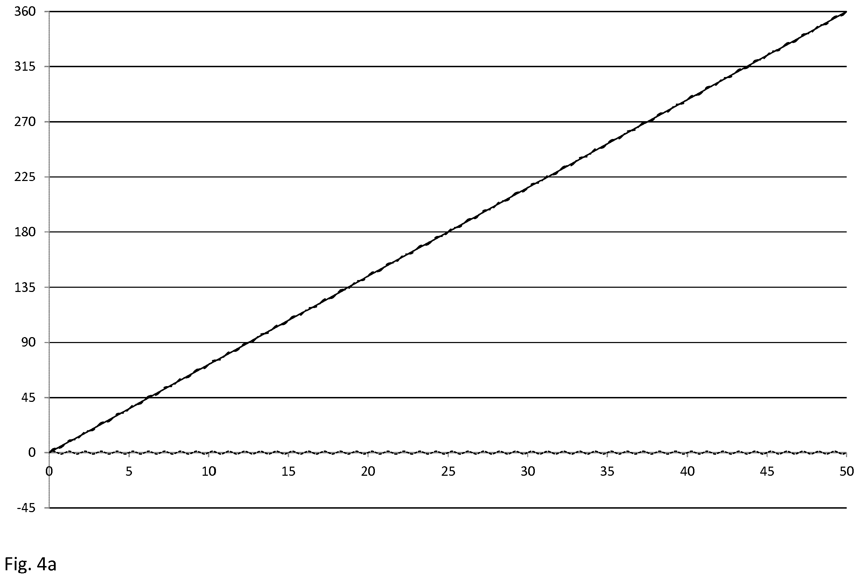

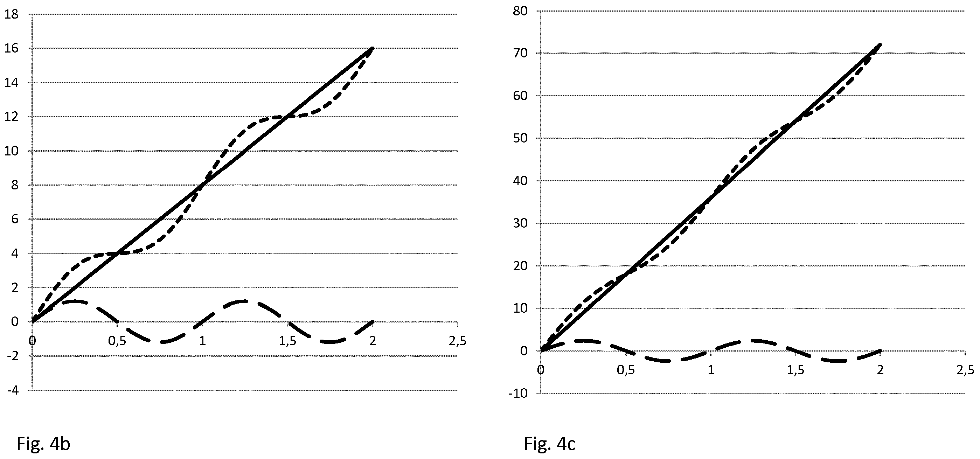

Figuren 4a bis 4c verdeutlichen die Problematik einer nicht exakten Parallelverschiebung des Antriebsrads 11 während der zweidimensionalen Schwingung. In dem in Fig. 4a gezeigten Diagramm ist der Drehwinkel des Abtriebsrads 10 als Ordinate gegenüber der Anzahl der Umläufe des Antriebsrads 11 aufgetragen. Eine exakte Parallelverschiebung ohne überlagerte rotatorische Bewegung des Antriebsrads 11 ergibt eine Ursprungsgerade, deren Steigung durch das Verhältnis des exzentrischen Versatzes $R_a - R_i$ zum Außendurchmesser $R_a$ des Abtriebsrads 10 festgelegt ist. In dem gezeigten Beispiel sind das Antriebsrad 11 und das Abtriebsrad 10 miteinander verzahnt, wobei die Zähnezahl $Z_1$ des Abtriebsrads 50 beträgt und die Zähnezahl $Z_2$ des Abtriebsrads 51 beträgt. Dadurch wird das Abtriebsrad 10 um einen Zahn pro Umlaufbewegung des Antriebsrads 11 weitergedreht bzw. für eine ganze Umdrehung des Abtriebsrads 10 um 360° bedarf es 50 Umläufe des Antriebsrads 11. Bei dem hier gewählten Gelenksystem 13 mit bestimmten Hebelverhältnissen und Abständen von Gelenkachsen ergibt sich eine rotatorische Schwingbewegung mit einer Amplitude von etwa 1,2°, die in Fig. 4a entlang der Abszisse dargestellt ist. Diese rotatorische Bewegung des Antriebsrads 11 wirkt sich auf das übertragene Drehmoment aus und überlagert sich der als Ursprungsgerade dargestellten rein translatorischen Bewegung (Parallelverschiebung). In Fig. 4b ist Fig. 4a für die ersten zwei Umläufe des Antriebsrads 11 im Detail gezeigt. Dabei wird deutlich, dass diese überlagerte rotatorische Bewegung durchaus erheblichen Einfluss auf den Antrieb des Abtriebsrads 10 hat. Die gestrichelt dargestellte Resultierende entspricht keiner kontinuierlichen Drehbewegung, sondern entspricht eher einer stufenartigen Drehbewegung. Prinzipiell ist jedoch eine möglichst gleichmäßige Drehbewegung, die einer Ursprungsgeraden am nächsten kommt, bevorzugt. Dies kann durch entsprechende Dimensionierung des Gelenksystems 13 mit bestimmten Hebelverhältnissen und Abständen von Gelenkachsen erzielt werden. In Fig. 4c ist beispielsweise eine Zähnezahl $Z_1$=10 des Abtriebsrads 10 und eine Zähnezahl $Z_2$=22 des Antriebsrads 11 gewählt sowie ein exzentrischer Versatz von 0,25 mm. Durch die kleineren Zähnezahlen und die größere Zähnezahldifferenz werden lediglich 10 Umlaufbewegungen des Antriebsrads 11 und damit Schwingungsperioden benötigt, um eine ganze Umdrehung des Abtriebsrads 10 um 360° zu realisieren. Durch das geänderte Untersetzungsverhältnis $(Z_2 - Z_1)/Z_1$ ist die Steigung der Ursprungsgeraden größer als im Beispiel der Fig. 4a und 4b. Wenngleich die Amplitude der rotatorischen Kippbewegung des Antriebsrads 11 hier größer ist, ist die Resultierende (gestrichelt dargestellt) weniger treppenartig und näher an der optimalen Ursprungsgeraden. Durch geeignete Dimensionierungen des Gelenksystems 13 kann also eine Parallelverschiebung erzielt werden, bei der eine überlagerte rotatorische Bewegung vernachlässigt werden kann.

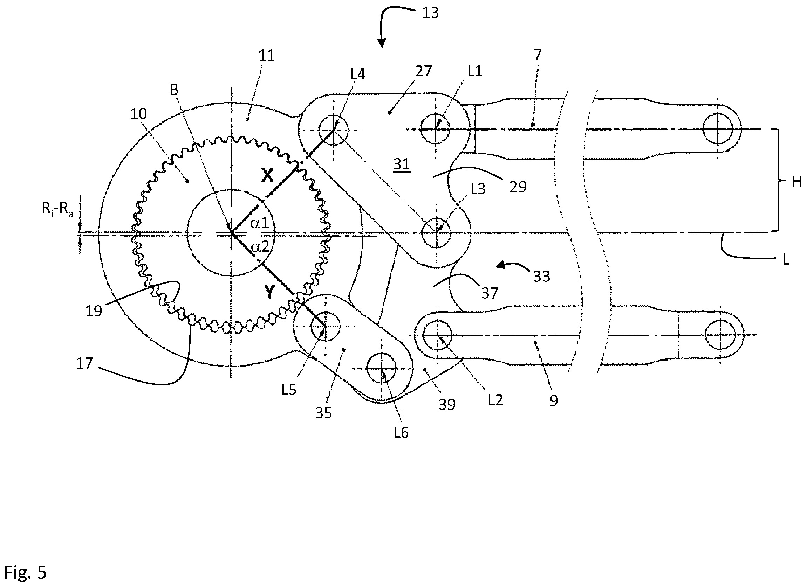

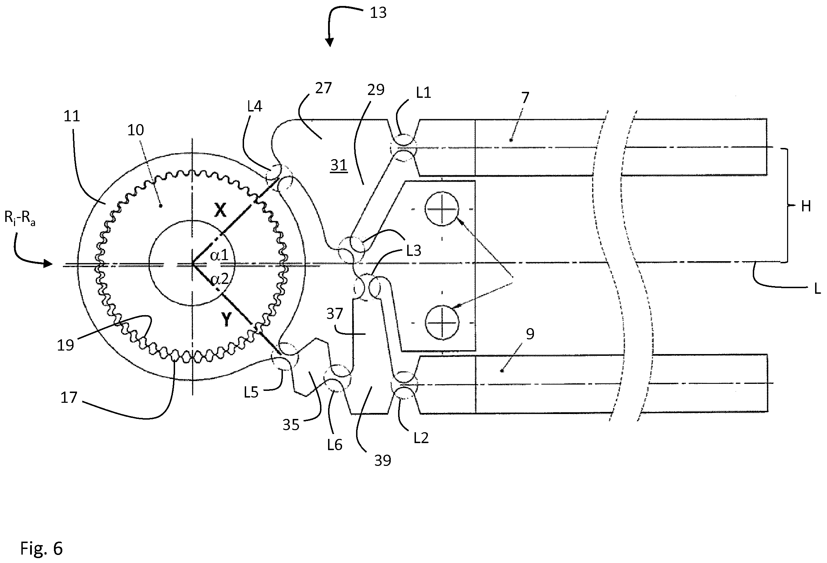

Fig. 5 und 6 zeigen zwei Ausführungsbeispiele, bei denen das Gelenksystem 13 so ausgelegt ist, dass die überlagerte rotatorische Bewegung des Antriebsrads 11 vernachlässigt werden kann. Die Ausführungsbeispiele der Fig. 5 und 6, die nicht Teil der Erfindung sind, unterscheiden sich dadurch, dass in Fig. 5 Hebel über gelagerte Gelenkachsen miteinander gekoppelt sind, und in Fig. 6 verformbare Festkörpergelenke die Beweglichkeit zwischen den Hebeln ermöglichen.

In Fig. 5 ist ein Abtriebsrad 10 gezeigt, das eine radiale Außenfläche 17 mit einer zykloidischen Verzahnung mit 50 Zähnen aufweist. Das Abtriebsrad 10 ist bezüglich eines hier nicht gezeigten Schafts 3 ortsfest um die Drehachse B drehbar gelagert. Das Abtriebsrad 10 ist von einem exzentrisch umgreifenden Antriebsrad 11 umgeben, das eine radiale Innenfläche 17 mit einer entsprechenden zykloidischen Verzahnung mit 51 Zähnen aufweist. Der exzentrische Versatz entspricht hier im Wesentlichen der Differenz zwischen Radius $R_a$ der radialen Außenfläche 19 des Abtriebsrads 10 und dem Radius $R_i$ der radialen Innenfläche 17 des Antriebsrads 11. Die Zahnhöhe der Verzahnungen ist nur ein wenig kleiner gewählt als der exzentrische Versatz $R_i - R_a$. Die bezüglich des Schafts 3 feste Drehachse B erstreckt sich orthogonal zur Längsachse L des Schafts 3 und schneidet diese. Das Antriebsrad 11 ist über ein Gelenksystem 13 mechanisch an zwei Betätigungselemente 7, 9 gekoppelt. Die Betätigungselemente 7, 9 sind in Form von länglichen Zug-/Schubstangen ausgestaltet und erstrecken sich innerhalb des Schafts 3 in dessen Längsrichtung L. Die beiden Betätigungselemente 7, 9 verlaufen symmetrisch zur Längsachse L lateral mit einem Abstand H zur Längsachse L versetzt und diametral gegenüberliegend bezüglich der Längsachse L. Die Betätigungselemente 7, 9 sind in Längsrichtung axial beweglich und jeweils mit ihrem distalen Ende über bewegliche Lagerachsen L1, L2 an das Gelenksystem 13 gekoppelt. Das Gelenksystem 13 ist selbst über eine bezüglich des Schafts 3 ortsfeste Lagerachse L3 gelagert, die parallel zur Drehachse B verläuft und versetzt zur Drehachse B die Längsachse L schneidet. Das Gelenksystem 13 ist über bewegliche Lagerachsen L4, L5 an das Antriebsrad 11 gekoppelt. Das Gelenksystem 13 weist einen ersten Hebel 27 auf, der die Lagerachse L1 mit der Lagerachse L3 verbindet. Das Gelenksystem 13 weist außerdem einen ersten Kopplungshebel 29 auf, der die Lagerachse L1 mit der ortsfesten Lagerachse L3 verbindet. Der erste Hebel 27 und der erste Kopplungshebel 29 verlaufen orthogonal zueinander als Teil eines einstückigen oberen Abschnitts 31 des Gelenksystems 13. Der obere Abschnitt 31 des Gelenksystems 13 legt die relative Position der Lagerachsen L1, L3 und L4 derart fest, dass diese in einem gleichschenkligen und rechtwinkligen Dreieck angeordnet sind, wobei der Abstand der Achsen L1 und L4 dem Abstand der Achsen L1 und L3 entspricht. Die Lagerachse L4 ist in ihrer Bewegung daher auf eine Kreisbahn um die feste Lagerachse L3 beschränkt. Bei einer axialen Bewegung des oberen Betätigungselements 7 und somit der Lagerachse L1 in tangentiale Richtung um die Lagerachse L3 wird das Antriebsrad 11 über die Lagerachse L4 in x-Richtung bewegt, wobei die x-Richtung einen ersten Winkel $\alpha1=45°$ mit der Längsachse L bildet. Wenn das Betätigungselement 7 also axial in Richtung der Längsachse L schwingt, so schwingt das Antriebsrad 11 in x-Richtung. Die Schwingungsamplitude, die sich in der Größenordnung des exzentrischen Versatzes $R_i - R_a$ liegt, ist so klein gegenüber dem Abstand der Lagerachsen L4 und L3, dass die überlagerte rotatorische Bewegung des Antriebsrads 11 vernachlässigbar klein ist. Ein unterer Abschnitt 33 des Gelenksystems 13 zur Ankopplung des distalen Endes des unteren Betätigungselements 9 über die Lagerachse L2 weist einen zweiten Hebel 35 und einen zweiten Kopplungshebel 37 auf. Der untere Abschnitt 33 ist nicht wie der obere Abschnitt 31 einstückig ausgebildet, sondern zweistückig mit einer zusätzlichen beweglichen Lagerachse L6 und einem Steuerhebel 39. Der zweite Kopplungshebel 37 ist im Wesentlichen symmetrisch bezüglich der Längsachse L zum ersten Steuerhebel 29 angeordnet und verbindet die bewegliche Lagerachse L2 mit der festen Lagerachse L3. Der Steuerhebel 39 und der zweite Kopplungshebel 37 spannen ein von den Lagerachsen L3, L2 und L6 definiertes Dreieck auf. Der zweite Hebel 35 verbindet dann die Lagerachse L5 und die Lagerachse L6 miteinander und koppelt somit den unteren Abschnitt 33 des Gelenksystems 13 an das Antriebsrad 11. Die axiale Bewegung des unteren Betätigungselements 9 in Längsrichtung L wird somit auf eine Bewegung der Lagerachse L5 in y-Richtung übertragen, wobei die y-Richtung einen zweiten Winkel $\alpha2=45°$ mit der Längsachse L bildet. Die Schwingungsrichtungen des Antriebsrads 11 in x-Richtung und x-Richtung verlaufen daher orthogonal zueinander. Schwingen die Betätigungselemente 7,9 nun um 90° phasenverschoben zueinander, so folgt das Antriebsrad im Wesentlichen parallelverschoben einer Kreisbahn, wobei der Radius der Kreisbahn im Wesentlichen dem exzentrischen Versatz $R_i - R_a$ entspricht. Dabei wird bei jeder Schwingungsperiode das Abtriebsrad 10 um einen Zahn weiter um die feste Drehachse B gedreht. Nach 50 Umläufen des Antriebsrads 11 bzw. Schwingungsperioden der Betätigungselemente 7, 9 hat das Abtriebsrad 10 dann genau eine ganze Umdrehung um 360° vollführt.

Die Festkörpergelenke in Fig. 6 entsprechen im Wesentlichen den Lagerachsen L1 bis L6 aus Fig. 5, wobei hier die feste Lagerachse L3 aus Fig. 5 durch zwei ortsfeste Festkörpergelenke gebildet ist, sodass das Gelenksystem 13 hier sieben Festkörpergelenke aufweist. Die Festkörpergelenke sind hier als verdünnte Abschnitte eines verformbaren Materials ausgebildet. Die Kinematik des Antriebsrads 11 und des Abtriebsrads 10 sind im Wesentlichen identisch zu dem in Fig. 5 dargestellten Ausführungsbeispiel. Ein Vorteil der in Fig. 5 und 6 dargestellten Ausführungsbeispiele, die nicht Teil der Erfindung sind, ist, dass die Bewegung eines Betätigungselements 7, 9 unabhängig von der Bewegung des anderen Betätigungselements 9, 7 ist und diese daher sehr einfach angesteuert werden können.

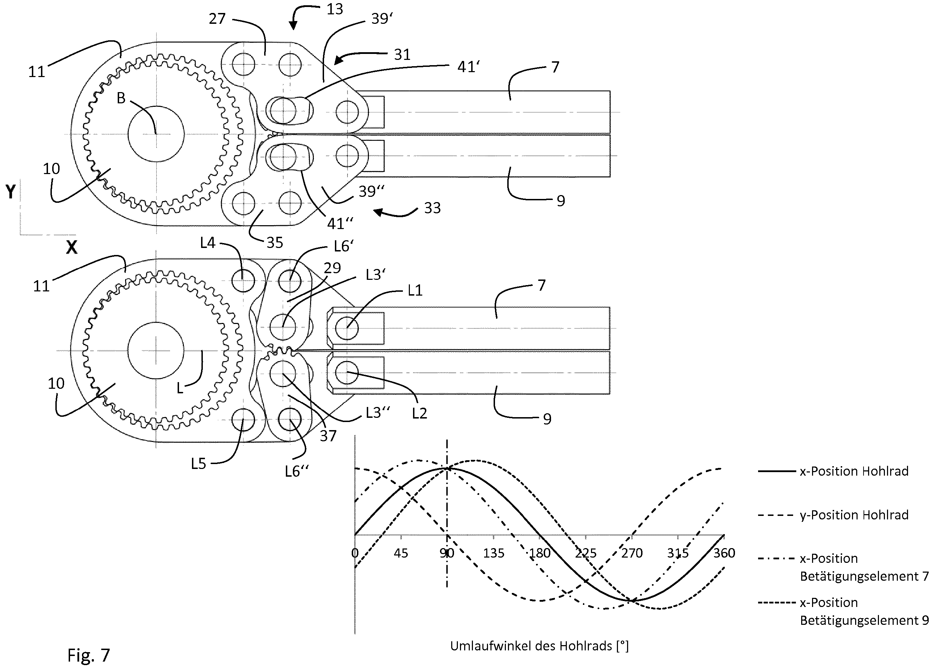

Dies ist in der in Fig. 7 dargestellten erfindungsgemäßen dritten Ausführungsform anders. Hier sind die Betätigungselemente 7, 9 derart miteinander gekoppelt, dass die Ansteuerung nur eines der Betätigungselemente 9, 7 unabhängig vom anderen Betätigungselement 9, 7 nicht möglich ist. Die Betätigungselemente 7, 9 müssen daher beide koordiniert angesteuert werden. Die komplexere Ansteuerung wird allerdings in Kauf genommen, um einen symmetrischen Aufbau des Gelenksystems 13 und somit eine reduzierte Bauteilevielfalt zu erzielen. Außerdem ist das Untersetzungsverhältnis bei dem in Fig. 7 gezeigten dritten Ausführungsbeispiel anders. Das Abtriebsrad 10 hat hier eine Zähnezahl $Z_1=40$. Das Antriebsrad 11 hat dagegen eine Zähnezahl $Z_2=44$, sodass sich ein Untersetzungsverhältnis von $Z_1/(Z_2-Z_1)=10$ ergibt. Es bedarf also zehn ganzer Umläufe des Antriebsrads 11, um das Abtriebsrad 10 um eine ganze Umdrehung zu drehen. Entsprechend sind die Radien $R_i$ und $R_a$ der radialen Innenfläche 17 des Antriebsrads 11 bzw. der radialen Außenfläche 19 des Abtriebsrads 10 so gewählt, dass sich ein Untersetzungsverhältnis $R_a/(R_i-R_a)=10$ ergibt, sodass ein Umlauf des Antriebsrads 11 das Abtriebsrad 10 um 36° bzw. vier Zähne weiterdreht. Das Gelenksystem 13 ist hier im Wesentlichen symmetrisch bezüglich der Längsachse L ausgestaltet, d.h. der obere Abschnitt 31 des Gelenksystems 13 ist symmetrisch und im Wesentlichen baugleich zum unteren Abschnitt 33 des Gelenksystems 13 aufgebaut. Das Gelenksystem 13 weist hier zwei bezüglich des hier nicht gezeigten Schafts 3 ortsfeste Lagerachsen L3' und L3'' auf. Die festen Lagerachsen L3' und L3'' sind symmetrisch bezüglich der Längsachse L angeordnet. Analog zu dem in Fig. 5 gezeigten Ausführungsbeispiel bilden hier Lagerachsen L1 und L2 die Ankopplung des Gelenksystem 13 an die distalen Enden der Betätigungselemente 7, 9 und Lagerachsen L4 und L5 die Ankopplung an das Antriebsrad 11. Das Gelenksystem 13 weist ferner Lagerachsen L6' und L6'' auf, die jeweils denselben Abstand zur Längsachse L haben wie die Lagerachse L4 bzw. L5, sodass sich zwischen der Lagerachse L4 und der Lagerachse L6' ein erster Hebel 27 ergibt, der parallel zur Längsachse L ausgerichtet ist. Analog ist zwischen der Lagerachse L5 und der Lagerachse L6'' ein zweiter Hebel 35 angeordnet, der sich ebenfalls parallel zur Längsachse L erstreckt. Zwischen der Lagerachse L6' und der Lagerachse L1 erstreckt sich ein erster Steuerhebel 39', und zwischen der Lagerachse L6'' und der Lagerachse L2 erstreckt sich ein zweiter Steuerhebel 39''. Die Lagerachsen L4, L6' und L1 sind relativ zueinander in einem Dreieck positioniert. Der erste Hebel 27 und der erste Steuerhebel 39' stehen in einem festen Winkel zueinander. Analog steht der zweite Hebel 35 in einem festen Winkel zum Steuerhebel 39''. Das Gelenksystem 13 weist sowohl im oberen Abschnitt 31 als auch im unteren Abschnitt 33 jeweils eine Schlitzführung 41', 41'' auf, die einem Kreisabschnitt folgt. Die obere Schlitzführung 41' folgt einem Kreisabschnitt um die obere Lagerachse L6', dessen Radius dem Abstand der Achse L6' von der festen Lagerachse L3' entspricht. Analog dazu folgt die untere Schlitzführung 41'' einem Kreisabschnitt um die untere Lagerachse L6'' mit einem Radius, der dem Abstand zwischen der unteren Lagerachse L6'' und der unteren festen Lagerachse L3'' entspricht. Durch die Schlitzführungen 41' und 41'' sind der erste Hebel 27 und der zweite Hebel 35 im Wesentlichen in x-Richtung beweglich, sodass sich das Antriebsrad in x-Richtung bewegt, wenn die Betätigungselemente 7, 9 gleich gerichtet bewegt werden. In der unteren Abbildung von Fig. 7 sind Kopplungshebel 29, 37 besser erkennbar, die sich von der festen Lagerachse L3' zur Lagerachse L6' bzw. von der festen Lagerachse L3'' zur Lagerachse L6'' erstrecken. Die Kopplungshebel 29, 37 sind derart miteinander verzahnt, dass die Lagerachsen L6' und L6'' immer symmetrisch zur Längsachse L angeordnet sind. Dadurch sind auch die Lagerachsen L4 und L5 immer an gleicher x-Position. Dadurch wird eine überlagerte rotatorische Bewegung des Antriebsrads 11 ausgeschlossen, sodass das Hohlrad 11 exakt parallelverschoben wird. Um das Antriebsrad 11 in y-Richtung zu bewegen, müssen die Betätigungselemente 7, 9 relativ zueinander bewegt werden. Wegen der Schlitzführungen 41' und 41'' werden die Lagerachsen L4 und L5 um die Lagerachsen L6' und L6'' gedreht, wobei sich die y-Position des Antriebsrads 11 verändert. Bewegt sich das obere Betätigungselement 7 beispielsweise in negative x-Richtung und das untere Betätigungselement 9 gleich schnell in positive x-Richtung, so bewegt sich das Antriebsrad 11 in negative y-Richtung, also nach unten, und bewegt sich nicht in x-Richtung. Im umgekehrten Fall, wenn sich das obere Betätigungselement 7 in positive x-Richtung bewegt und das untere Betätigungselement 9 gleich schnell in negative x-Richtung, so bewegt sich das Antriebsrad 11 nur in positive y-Richtung rein parallelverschoben nach oben. Hierbei kommt es nur auf die relative Bewegung der Betätigungselemente 7 und 9 zueinander an. Das heißt, wenn sich beide Betätigungselemente 7, 9 in x-Richtung bewegen, allerdings das obere Betätigungselement 7 schneller als das untere Betätigungselement 9, so bewegt sich das Antriebsrad 11 sowohl in x-Richtung als auch in positive y-Richtung nach oben, da sich das obere Betätigungselement 7 relativ zum unteren Betätigungselement 9 nach rechts in positive x-Richtung bewegt.

Das Phasendiagramm in Fig. 7 verdeutlicht die Bewegungen des Hohlrads in x- und y-Richtung und die Bewegungen der Betätigungselemente 7, 9 in x-Richtung bezüglich des Umlaufwinkels des Hohlrads in Grad. In Fig. 7 ist ein Umlaufwinkel des Hohlrads von 90° gezeigt, bei dem die y-Position des Hohlrads 0 ist und die x-Position maximal, d. h. gleich dem exzentrischen Versatz $R_a - R_i$. Wie aus dem Diagramm zu erkennen ist, schwingen die Betätigungselemente 7,9 mit einer um den Faktor $\sqrt{1,25}$ größeren Amplitude in x-Richtung. Außerdem ist die Schwingung des Betätigungselements 7 um einen Phasenwinkel arctan(0,5)=29,5° nach links phasenverschoben zur x-Position des Hohlrads. Das untere Betätigungselement 9 ist entsprechend um den Phasenwinkel arctan(0,5)=29,5° nach rechts phasenverschoben bezüglich der x-Position des Antriebsrads 11. Die Ansteuerung der Betätigungselemente 7, 9 erfolgt also genau und konzertiert entlang der dargestellten Kurven, um die entsprechende exakt parallelverschobene Kreisbahn des Antriebsrads 11 um das Abtriebsrad 10 herum zu erzielen.

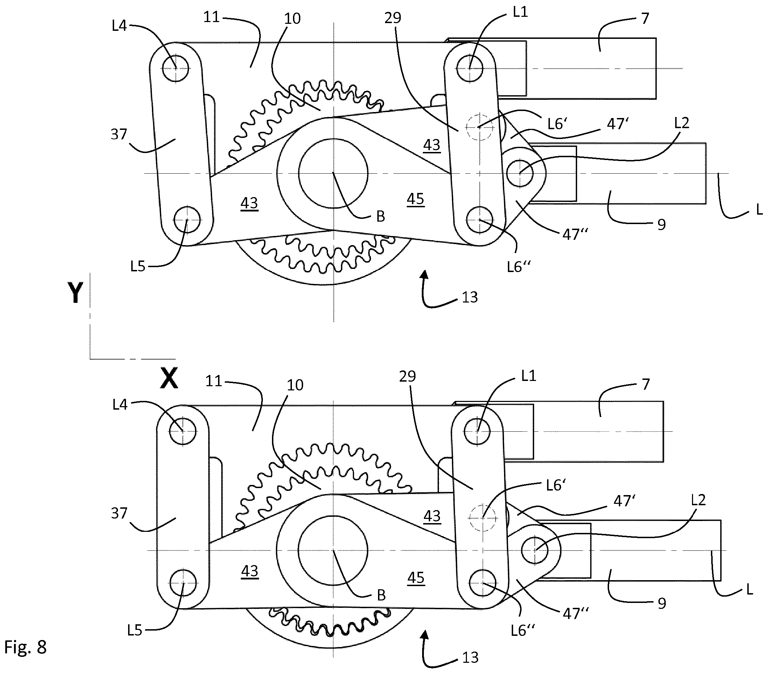

In Fig. 8 wird ebenfalls eine exakt parallelverschobene Kreisbahn des Antriebsrads 11 erzielt. Hierbei weist das Gelenksystem 13 einen Doppelhebel 43 und einen Hebel 45 auf, die jeweils drehbar um die ortsfeste Drehachse B des Abtriebsrads 10 angeordnet sind. Der Doppelhebel 43 ist symmetrisch bezüglich der Drehachse B aufgebaut. Der Hebel 45 ist gleich dimensioniert wie eine Hälfte des Doppelhebels 43. An den beweglichen Lagerachsen L6' und L6" sind zwei Kniehebel 47', 47" mit dem Doppelhebel 43 bzw. mit dem Hebel 45 verbunden, wobei die Kniehebel 47', 47" wiederum über die Lagerachse L2 mit dem Betätigungselement 9 verbunden sind. Das Betätigungselement 9 ist in x-Richtung verschiebbar und in die übrigen Raumrichtungen ortsfest gelagert. In den Lagerachsen L6" und L5 sind mit dem Doppelhebel 43 bzw. mit dem Hebel 45 jeweils ein Kopplungshebel 29, 37 verbunden, welche die oberen Lagerachsen L1 und L4 aufweisen. In diesen Lagerachsen L1 und L4 ist das Antriebsrad 11 mit den Kopplungshebeln 29, 37 verbunden. In der Lagerachse L1 greift zudem das obere Betätigungselement 7 an das Hohlrad 11 an. Durch den symmetrischen Aufbau des Doppelhebels 43, der Kopplungshebel 29, 37 sowie die ortsfeste Lage des unteren Betätigungselements 9 werden die Lagerachsen L6" und L5 stets parallel, d.h. mit gleicher y-Position, geführt. Eine Betätigung des Betätigungselements 9 in x-Richtung bewegt über die Kniehebel 47', 47" eine simultane Verschiebung der Lagerachsen L5 und L6" in y-Richtung. Da die Kopplungshebel 29, 37 identisch sind, bewegen sich dabei auch die Lagerachsen L1 und L4 simultan zueinander in y-Richtung. Da die Kopplungshebel 29, 37 um die Lagerachsen L5 und L6" drehbar gelagert sind, kann über das Betätigungselement 7 eine Bewegung des Hohlrads 11 in x-Richtung eingeleitet werden. Durch diese Parallelkinematik des Gelenksystems 13 wird eine Überlagerung einer Drehbewegung vollständig vermieden. Das Antriebsrad 11 bewegt sich bei der Ansteuerung der Betätigungselemente 7, 9 daher rein translatorisch in x- und/oder y-Richtung. Bei einer entsprechenden sinusähnlichen und phasenverschobenen Anlenkung durch beide Betätigungselemente 7, 9 kann das Hohlrad 11 eine exakte Kreisbahn ohne überlagerte Verdrehung ausführen. Allerdings tritt durch den Einsatz der Kniehebel 47', 47" ein nichtlinearer Zusammenhang der Bewegung des Betätigungselements 9 in x-Richtung und der y-Verschiebung des Antriebsrads 11 auf.

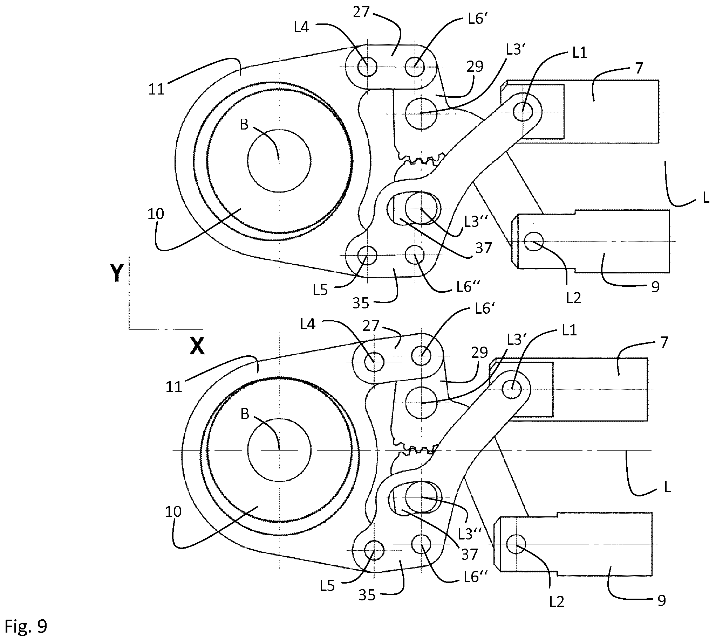

In Fig. 9 ist eine weitere Ausführungsform dargestellt, die nicht Teil der Erfindung ist. Bei dieser Ausführungsform wird die Parallelverschiebung des Hohlrads 11 durch eine Bewegungskopplung der Kopplungshebel 29, 37 sowie die Anbindung des Antriebsrads 11 an die Kopplungshebel 29, 37 über die Hebel 27, 35 erreicht.

Der Kopplungshebel 29 ist drehbar um die ortsfeste Lagerachse L3' und der Kopplungshebel 37 entsprechend um die ortsfeste Lagerachse L3" gelagert. Beide Kopplungshebel 29, 37 sind über Verzahnungsabschnitte miteinander gekoppelt, wobei das Übersetzungsverhältnis hier 1:1 ist. An dem Kopplungshebel 29 befindet sich eine Lagerachse L6' und an dem Kopplungshebel 39 entsprechend eine Lagerachse L6", wobei die Achsabstände von L6' zu L3' und L6" zu L3" gleich sind. Dadurch bewegen sich die Lagerachsen L6' und L6" immer simultan auf Kreisbögen, im Wesentlichen aber nur in x-Richtung. An dem als Doppelhebel ausgeführten Kopplungshebel 29 ist an der Lagerachse L2 das untere Betätigungselement 9 sowie an der Lagerachse L6' der Hebel 27 drehbar gelagert. Entsprechend ist an dem Kopplungshebel 37 der Hebel 35 über die Lagerachse L6" drehbar gelagert. Der Hebel 27 besitzt eine weitere bewegliche Lagerachse L4, an welcher das Hohlrad 11 drehbar gelagert ist. Am ebenfalls als Doppelhebel ausgeführten Hebel 35 befinden sich die Lagerachsen L1, an welcher das obere Betätigungselement 7 gelagert ist, sowie die Lagerachse L5, an welcher das Hohlrad 11 drehbar gelagert ist. Die Achsabstände von Lagerachse L6' zu L4 bzw. L6" zu L5 sind gleich. Wird eine Bewegung über das untere Betätigungselement 9 eingeleitet, so dreht sich der Kopplungshebel 29 um die Achse L3'. Durch die Verzahnung der Kopplungshebel 29, 37 befinden sich die Lagerachsen L6' und L6" immer auf gleicher x-Position. Wird eine Bewegung über das obere Betätigungselement 7 eingeleitet, so dreht sich der Hebel 35 um die Achse L6". Die Lagerachse L5 bewegt sich dabei bogenförmig um die Lagerachse L6", im Wesentlichen aber in y-Richtung. Durch die beschriebene Parallelkinematik kann das Hohlrad 11 rein translatorisch in x- und/oder y-Richtung bewegt werden. Durch eine Einleitung von entsprechenden Bewegungen an den Betätigungselementen 7, 9 kann das Hohlrad 11 rein translatorisch auf einer Kreisbahn um die Drehachse B bewegt werden. Die Bewegungen der Betätigungselemente 7, 9 sind hier allerdings gekoppelt wie in Fig. 7.

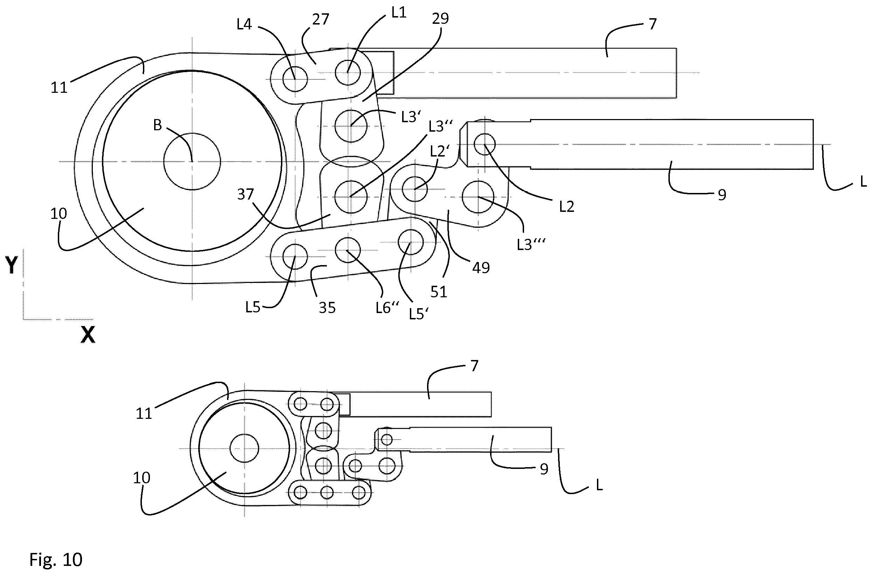

In Fig. 10 ist eine weitere Ausführungsform dargestellt, die nicht Teil der Erfindung ist. Hier sind die Kopplungshebel 29, 37 auf den ortsfesten Achsen L3' und L3" drehbar gelagert und durch eine Verzahnung mittels korrespondierender Verzahnungsabschnitte (nicht detailliert dargestellt) miteinander gekoppelt. Der Kopplungshebel 29 weist eine weitere Lagerachse L1 auf, an der der Hebel 27 sowie das obere Betätigungselement 7 drehbar sind. Der Kopplungshebel 37 weist eine weitere Lagerachse L6" auf, an der ein symmetrischer Doppelhebel 35 drehbar gelagert ist. Am Hebel 27 und am Doppelhebel 35 ist jeweils über die Lagerachsen L4 bzw. L5 das Hohlrad 11 drehbar gelagert. Eine Bewegung des Betätigungselements 7 bewirkt eine Verdrehung des Kopplungshebels 29 um die ortsfeste Lagerachse L3' und, da die Verzahnungsabschnitte ineinander greifen, auch eine Verdrehung des Kopplungshebels 37 im gleichen Maße in entgegengesetzte Richtung um L3".

Die Lagerachsen L6' und L6" befinden sich somit jederzeit auf gleicher x-Position. Da die Achsabstände L1 zu L4 und L6" zu L5 gleich sind, befinden somit auch die Lagerachsen L4 und L5 jederzeit auf gleicher x-Position. Durch eine Bewegung des oberen Betätigungselements 7 wird also eine translatorische Bewegung des Antriebsrads 11 in x-Richtung eingeleitet. Bei einer Bewegung des unteren Betätigungselements 9 wird ein Umlenkhebel 49 gedreht, welcher um die ortsfeste Achse L3‴ gelagert ist. Ein Verbindungshebel 51 ist an dem Umlenkhebel 49 in der Lagerachse L2' sowie an einer weiteren Lagerachse L5' des Hebels 35 drehbar gelagert und überträgt die Bewegung der Lagerachse L2' im Wesentlichen in y-Richtung auf den symmetrischen Doppelhebel 35, diese Bewegung auf die Lagerachse L5 in y-Richtung überträgt. Eine Bewegung des unteren Betätigungselements 9 erzeugt daher eine translatorische Bewegung des Antriebsrads 11 in y-Richtung. Der Vorteil dieser Ausführungsform liegt in den weitgehend entkoppelten Bewegungen der Betätigungselemente 7 und 9 für die Bewegung des Hohlrads 11 in x- und y-Richtung. Bei einer entsprechenden phasenverschobenen sinusförmigen Bewegungseinleitung in die Betätigungselemente 7 und 9 kann das Hohlrad rein translatorisch auf einer Kreisbahn um die Drehachse B bewegt werden.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Als optional, vorteilhaft, bevorzugt, erwünscht oder ähnlich bezeichnete "kann"-Merkmale sind als optional zu verstehen.

Die beschriebenen Ausführungsformen sind als illustrative Beispiele zu verstehen und stellen keine abschließende Liste von möglichen Ausführungsformen dar.


## Patentansprüche

1.  Längliches Schaftinstrument (1) mit einem Antriebssystem mit

    - zwei sich im Wesentlichen parallel zueinander in einer Längsrichtung (L) oszillatorisch beweglichen Impulsgebern (23, 25),
    - einem um eine Drehachse (B) drehbar gelagerten Abtriebsrad (10) zum Antreiben einer Bewegung und/oder Betätigung eines distalseitigen Bauteils oder Instrumentenkopfs (5) des Schaftinstruments (1), und
    - einem translatorisch begrenzt beweglichen Antriebsrad (11) zum Kraft-, Form- und/oder Reibschluss mit dem Abtriebsrad (10),
wobei das Antriebsrad (11) durch die zwei Impulsgeber (23, 25) mit zwei länglichen Betätigungselementen (7, 9) antreibbar in zwei Oszillationsrichtungen (x, y) oszillatorisch beweglich ist,

**dadurch gekennzeichnet, dass**
die Betätigungselemente (7, 9) jeweils distalseitig über ein Gelenksystem (13) mit Lagerachsen (L1, L2) mechanisch an das Antriebsrad (11) gekoppelt sind, wobei das Gelenksystem (13) zwei ortsfeste Lagerachsen (L3', L3"), zwei bewegliche Lagerachsen (L6', L6"), zwei Steuerhebel (39', 39"), zwei Lagerachsen (L4, L5) zur Ankopplung des Gelenksystems (13) an das Antriebstrad (11), zwei jeweils parallel zur Längsache (L) ausgerichtete Hebel (27, 35), die sich jeweils zwischen einer der beiden Lagerachsen (L4, L5) und einer der beweglichen Lagerachsen (L6', L6") ergeben und wobei die Hebel (27, 35) jeweils in einem festen Winkel zu einem der Steuerhebel (39', 39") stehen, zwei Kopplungshebel (29, 37) und zwei Schlitzführungen (41', 41") aufweist, wobei die jeweilige Schlitzführung (41', 41") einem Kreisabschnitt um die jeweilige bewegliche Lagerachse (L6', L6") folgt, dessen Radius dem Abstand der beweglichen Lagerachse (L6', L6") von der festen Lagerachse (L3', L3") entspricht, so dass die jeweiligen Hebel (27, 35) jeweils im Wesentlichen in Längsrichtung (L) beweglich sind und dass die Lagerachsen (L4, L5) zur Ankopplung des Gelenksystems an das Antriebsrad um die jeweilige bewegliche Lagerachse (L6', L6") drehbar sind, und wobei sich der jeweilige Steuerhebel (39', 39") von der jeweiligen Lagerachse (L1, L2) zur jeweiligen beweglichen Lagerachse (L6', L6") erstreckt, und wobei sich der jeweilige Kopplungshebel (29, 37) von der jeweiligen festen Lagerachse (L3', L3") zur jeweiligen beweglichen Lagerachse (L6', L6") erstreckt, wobei die zwei Kopplungshebel (29, 37) derart miteinander verzahnt sind, dass die Lagerachsen (L6', L6") immer symmetrisch zur Längsrichtung (L) angeordnet sind.

2.  Schaftinstrument (1) nach Anspruch 1, wobei die zwei Oszillationsrichtungen (x, y) zueinander im Wesentlichen orthogonal verlaufen.

3.  Schaftinstrument (1) nach Anspruch 1 oder 2, wobei das Abtriebsrad (10) durch einen abwälzenden Kraft-, Form- und/oder Reibschluss zwischen einer radialen Fläche (17) des Antriebsrads (11) und einer radialen Fläche (19) des Abtriebsrads (10) vom Antriebsrad (11) kontinuierlich antreibbar ist.

**4.** Schaftinstrument (1) nach einem der vorhergehenden Ansprüche, wobei das Antriebsrad (11) als ein das Abtriebsrad (10) exzentrisch umgreifendes Hohlrad ausgeführt ist.

**5.** Schaftinstrument (1) nach Anspruch 3 und 4, wobei die radiale Fläche (17) des Antriebsrads (11) eine radiale Innenfläche (17) und die radiale Fläche (19) des Abtriebsrads (10) eine radiale Außenfläche (19) ist.

**6.** Schaftinstrument (1) nach einem der Ansprüche 1 bis 3, wobei das Abtriebsrad als Hohlrad ausgeführt ist, wobei das Antriebsrad exzentrisch in das Abtriebsrad eingreift.

**7.** Schaftinstrument (1) nach einem der vorhergehenden Ansprüche, wobei die Impulsgeber (23, 25) dazu ausgestaltet sind, das Antriebsrad (11) im Wesentlichen 90° phasenverschoben zueinander oszillatorisch anzutreiben, sodass es einer Kreisbahn (21) im Wesentlichen parallelverschoben folgt, wobei der Radius der Kreisbahn im Wesentlichen einem exzentrischen Versatz zwischen dem Abtriebsrad (10) und dem Antriebsrad (11) entspricht.

**8.** Schaftinstrument (1) nach einem der vorhergehenden Ansprüche, wobei die Drehrichtung des Abtriebsrads (10) durch eine Phasenverschiebung zwischen den Impulsgebern (23, 25) bestimmt ist.

**9.** Schaftinstrument (1) nach einem der vorhergehenden Ansprüche, wobei das Antriebsrad (11) in eine erste (x) der Oszillationsrichtungen (x, y) oszillatorisch beweglich ist, wenn die Auslenkung der Impulsgeber (23, 25) in der Summe ungleich null ist, und wobei das Antriebsrad (11) in eine zweite (y) der Oszillationsrichtungen (x, y) oszillatorisch beweglich ist, wenn sich die Impulsgeber (23, 25) relativ zueinander bewegen.

**10.** Schaftinstrument (1) nach einem der vorhergehenden Ansprüche, wobei das Gelenksystem (13) eine im Wesentlichen in Längsrichtung (L) oszillierende Bewegung eines ersten (23) der zwei Impulsgeber (23, 25) um einen ersten Winkel ($\alpha$1) in eine erste (x) der Oszillationsrichtungen (x, y) des Antriebsrads (11) umlenkt.

**11.** Schaftinstrument (1) nach Anspruch 10, wobei das Gelenksystem (13) eine im Wesentlichen in Längsrichtung (L) oszillierende Bewegung eines zweiten (25) der zwei Impulsgeber (23, 25) in eine zweite (y) der Oszillationsrichtungen (x, y) des Antriebsrads (11) überträgt.

**12.** Schaftinstrument (1) nach Anspruch 10 oder 11, wobei das Gelenksystem (13) eine im Wesentlichen in Längsrichtung (L) oszillierende Bewegung eines zweiten (25) der zwei Impulsgeber (23, 25) um einen zweiten Winkel ($\alpha$2) in eine zweite (y) der Oszillationsrichtung (x, y) des Antriebsrads (11) umlenkt, wobei die Summe des ersten ($\alpha$1) und zweiten Winkels ($\alpha$2) im Wesentlichen 90° beträgt.

**13.** Schaftinstrument (1) nach Anspruch 12, wobei der erste Winkel ($\alpha$1) und der zweite Winkel ($\alpha$2) im Wesentlichen 45° betragen.

**14.** Schaftinstrument (1) nach einem der vorhergehenden Ansprüche, wobei die Kopplungshebel (29, 37) jeweils an mindestens der zur Drehachse (B) parallelen und ortsfesten Lagerachse (L3', L3") drehbar gelagert sind.

**15.** Schaftinstrument (1) nach einem der vorhergehenden Ansprüche, wobei die ortsfesten Lagerachsen (L3', L3") symmetrisch bezüglich einer von der Längsrichtung (L) und der Drehachse (B) aufgespannten Spiegelebene angeordnet sind.

**Claims**

**1.** An elongate shaft instrument (1) with a drive system comprising

   - two pulse generators (23, 25) oscillating substantially parallel to each other in a longitudinal direction (L),
   - an output wheel (10) rotatably mounted about an axis of rotation (B) for driving a movement and/or actuation of a distal component or instrument head (5) of the shaft instrument (1), and
   - a drive wheel (11) with limited translational movement for force-locking, form-fitting and/or integrally bonded connection to the output wheel (10),
wherein the drive wheel (11) can be driven by the two pulse generators (23, 25) with two elongate actuation elements (7, 9) and is oscillatable in two oscillation directions (x, y),
**characterised in that**
the actuation elements (7, 9) are each mechanically coupled to the drive wheel (11) on the distal side via a joint system (13) with bearing axes (L1, L2), wherein the joint system (13) has two stationary bearing axes (L3', L3''), two movable bearing axes (L6', L6"), two control levers (39', 39"), two bearing axes (L4, L5) for coupling the joint system (13) to the drive wheel (11), two levers (27, 35) each oriented parallel to the longitudinal axis (L), which are each formed between one of the two bearing axes (L4, L5) and one of the movable bearing axes (L6', L6''), and wherein the levers (27, 35) are each at a fixed angle to one of the control levers (39', 39"), two coupling levers (29, 37) and two slot guides (41', 41"), wherein the respective slot guide (41', 41") follows a circular portion around the respective movable bearing axis (L6', L6''), the radius of which corresponds to the distance of the movable bearing axis (L6', L6'') from the fixed bearing axis (L3', L3''), so that the respective levers (27, 35) are each movable substantially in the longitudinal direction (L), and **in that** the bearing axes (L4, L5) are rotatable about the respective movable bearing axis (L6', L6") to couple the joint system to the drive wheel, and wherein the respective control lever (39', 39") extends from the respective bearing axis (L1, L2) to the respective movable bearing axis (L6', L6''), and wherein the respective coupling lever (29, 37) extends from the respective fixed bearing axis (L3', L3'') to the respective movable bearing axis (L6', L6''), wherein the two coupling levers (29, 37) are toothed with one another in such a way that the bearing axes (L6', L6") are always arranged symmetrically to the longitudinal direction (L).

2. The shaft instrument (1) according to claim 1, wherein the two directions of oscillation (x, y) are substantially orthogonal to each other.

3. The shaft instrument (1) according to claim 1 or 2, wherein the output wheel (10) can be continuously driven by the drive wheel (11) by means of a rolling force, force-locking, form-fitting and/or frictional connection between a radial surface (17) of the drive wheel (11) and a radial surface (19) of the output wheel (10).

4. The shaft instrument (1) according to any one of the preceding claims, wherein the drive wheel (11) is designed as a ring gear eccentrically engaging around the output wheel (10)

5. The shaft instrument (1) according to claims 3 and 4, wherein the radial surface (17) of the drive wheel (11) is a radial inner surface (17) and the radial surface (19) of the output wheel (10) is a radial outer surface (19).

6. The shaft instrument (1) according to any one of claims 1 to 3, wherein the output wheel is designed as a ring gear, wherein the drive wheel engages eccentrically in the output wheel.

7. The shaft instrument (1) according to any one of the preceding claims, wherein the pulse generators (23, 25) are designed to drive the drive wheel (11) in an oscillatory manner substantially 90° out of phase with each other, so that it follows a circular path (21) substantially parallel displaced, wherein the radius of the circular path substantially corresponds to an eccentric offset between the output wheel (10) and the drive wheel (11).

8. The shaft instrument (1) according to any one of the preceding claims, wherein the direction of rotation of the output wheel (10) is determined by a phase shift between the pulse generators (23, 25).

9. The shaft instrument (1) according to any one of the preceding claims, wherein the drive wheel (11) is oscillatable in a first (x) of the oscillation directions (x, y) when the deflection of the pulse generators (23, 25) is not equal to zero in total, and wherein the drive wheel (11) is oscillatable in a second (y) of the oscillation directions (x, y) when the pulse generators (23, 25) move relative to each other.

10. The shaft instrument (1) according to any one of the preceding claims, wherein the joint system (13) transmits a movement of a first (23) of the two pulse generators (23, 25) oscillating substantially in the longitudinal direction (L) by a first angle (a1) into a first (x) of the oscillation directions (x, y) of the drive wheel (11).

11. The shaft instrument (1) according to claim 10, wherein the joint system (13) transmits a second (25) of the two pulse generators (23, 25) oscillating substantially in the longitudinal direction (L) into a second (y) of the oscillation directions (x, y) of the drive wheel (11).

12. The shaft instrument (1) according to claim 10 or 11, wherein the joint system (13) deflects a movement of a second (25) of the two pulse generators (23, 25) oscillating substantially in the longitudinal direction (L) by a second angle (a2) into a second (y) of the oscillation directions (x, y) of the drive wheel (11), wherein the sum of the first (a1) and second angle (a2) is substantially 90°.

13. The shaft instrument (1) according to claim 12, wherein the first angle (a1) and the second angle (a2) are substantially 45°.

14. The shaft instrument (1) according to any one of the preceding claims, wherein the coupling levers (29, 37) are each mounted rotatably on at least the stationary bearing axis (L3', L3'') parallel to the axis of rotation (B).

15. The shaft instrument (1) according to any one of the preceding claims, wherein the stationary bearing axes (L3', L3'') are arranged symmetrically with respect to a mirror plane spanned by the longitudinal direction (L) and the axis of rotation (B).

**Revendications**

1. Instrument à arbre (1) longitudinal avec un système d'entraînement comprenant :

   - deux générateurs d'impulsion (23, 25) mobiles de manière oscillatoire et essentiellement parallèles entre eux dans un sens longitudinal (L),
   - une roue de sortie (10) disposée rotative sur un pivot (B) pour entraîner un mouvement et/ou un actionnement d'une pièce côté distal ou d'une tête d'instrument (5) de l'instrument à arbre (1), et
   - une roue motrice (11) mobile délimitée en translation pour l'adhérence par force, par forme et/ou frottement avec la roue de sortie (10),

   dans lequel la roue motrice (11) est mobile en oscillation en pouvant être entraînée par les deux générateurs d'impulsion (23, 25) avec deux éléments d'actionnement (7, 9) longitudinaux dans deux directions d'oscillation (x, y), **caractérisé en ce que** les éléments d'actionnement (7, 9) sont respectivement couplés mécaniquement à la roue d'entraînement (11) côté distal par un système d'articulation (13) avec des axes de palier (L1, L2), dans lequel le système d'articulation (13) présente deux axes de palier fixes (L3', L3''), deux axes de palier mobiles (L6', L6''), deux leviers de commande (39', 39''), deux axes de palier (L4, L5) pour coupler le système d'articulation (13) à la roue motrice (11), deux leviers (27, 35) dirigés parallèles à l'axe longitudinal (L) qui sont produits respectivement entre un des deux axes de palier (L4, L5) et un des axes de palier mobiles (L6', L6'') et dans lequel les leviers (27, 35) sont respectivement dans un angle fixe par rapport à un des leviers de commande (39', 39''), deux leviers de couplage (29, 37) et deux guides fendus (41', 41''), dans lequel le guide fendu (41', 41'') respectif suit un segment circulaire autour de l'axe de palier mobile (L6', L6'') respectif, dont le rayon correspond à l'écart de l'axe de palier mobile (L6', L6'') par rapport à l'axe de palier fixe (L3', L3'') de sorte que les leviers (27, 35) respectifs sont mobiles respectivement essentiellement dans le sens longitudinal (L) et que les axes de palier (L4, L5) pour le couplage du système d'articulation sur la roue motrice sont rotatifs sur l'axe de palier mobile (L6', L6'') respectif, et dans lequel le levier de commande (39', 39'') respectif de l'axe de palier (L1, L2) respectif s'étend jusqu'à l'axe de palier mobile (L6', L6'') respectif, et dans lequel le levier de couplage (29, 37) respectif s'étend de l'axe de palier fixe (L3', L3'') respectif vers l'axe de palier mobile (L6', L6'') respectif, dans lequel les deux leviers de couplage (29, 37) respectifs sont ainsi engrenés entre eux que les axes de palier (L6', L6'') sont toujours disposés symétriquement à l'axe longitudinal (L).

2. Instrument à arbre (1) selon la revendication 1, dans lequel les deux sens d'oscillation (x, y) sont essentiellement orthogonaux entre eux.

3. Instrument à arbre (1) selon la revendication 1 ou 2, dans lequel la roue de sortie (10) peut être entraînée en continu par la roue motrice (11) par une adhérence de force, de forme et/ou de frottement par roulement entre une face radiale (17) de la roue motrice (11) et une face radiale (19) de la roue de sortie (10).

4. Instrument à arbre (1) selon l'une des revendications précédentes, dans lequel la roue motrice (11) est conçue en tant qu'une roue creuse entourant la roue de sortie (10) de manière excentrique.

5. Instrument à arbre (1) selon la revendication 3 et 4, dans lequel la face radiale (17) de la roue motrice (11) est une face intérieure radiale (17) et la face radiale (19) de la roue de sortie (10) est une face externe radiale (19).

6. Instrument à arbre (1) selon l'une des revendications 1 à 3, dans lequel la roue de sortie est conçue en tant que roue creuse, dans lequel la roue d'entraînement s'engrène de manière excentrique dans la roue de sortie.

7. Instrument à arbre (1) selon l'une des revendications précédentes, dans lequel les générateurs d'impulsion (23, 25) sont conçus pour entraîner en oscillation la roue d'entraînement (11) en décalage de phase essentiellement de 90° entre eux, de façon à ce qu'elle suive une trajectoire circulaire (21) essentiellement en décalage parallèle, dans lequel le rayon de la trajectoire circulaire correspond essentiellement à un décalage excentrique entre la roue de sortie (10) et la roue d'entraînement (11).

8. Instrument à arbre (1) selon l'une des revendications précédentes, dans lequel le sens de rotation de la roue de sortie (10) est déterminé par un décalage de phase entre les générateurs d'impulsion (23, 25).

9. Instrument à arbre (1) selon l'une des revendications précédentes, dans lequel la roue d'entraînement (11) est mobile de manière oscillatoire dans un premier (x) des sens d'oscillation (x, y), lorsque la flèche des générateurs d'impulsion (23, 25) dans la somme n'est pas égale à zéro, et dans lequel la roue d'entraînement (11) est mobile de manière oscillatoire dans un second (y) des sens d'oscillation (x, y), lorsque les générateurs d'impulsion (23, 25) se déplacent l'un par rapport à l'autre.

10. Instrument à arbre (1) selon l'une des revendications précédentes, dans lequel le système d'articulation (13) dévie un mouvement oscillatoire essentiellement dans le sens (L) d'un premier (23) des deux générateurs d'impulsion (23, 25) d'un premier angle (a1) dans un premier (x) des sens d'oscillation (x, y) de la roue d'entraînement (11).

11. Instrument à arbre (1) selon la revendication 10, dans lequel le système d'articulation (13) transfère un mouvement oscillatoire essentiellement dans le sens (L) d'un second (25) des deux générateurs d'impulsion (23, 25) dans un second (y) des sens d'oscillation (x, y) de la roue d'entraînement (11).

12. Instrument à arbre (1) selon la revendication 10 ou 11, dans lequel le système d'articulation (13) dévie un mouvement oscillatoire essentiellement dans le sens (L) d'un second (25) des deux générateurs d'impulsion (23, 25) d'un second angle (a2) dans un second (y) des sens d'oscillation (x, y) de la roue d'entraînement (11), dans lequel la somme du premier angle (a1) et du second angle (a2) fait essentiellement 90°.

13. Instrument à arbre (1) selon la revendication 12, dans lequel le premier angle (a1) et le second angle (a2) font essentiellement 45°.

14. Instrument à arbre (1) selon l'une des revendications précédentes, dans lequel les leviers de couplage (29, 37) sont disposés rotatifs respectivement sur au moins l'axe de palier (L3', L3'') parallèle et fixe par rapport au pivot (B).

15. Instrument à arbre (1) selon l'une des revendications précédentes, dans lequel les axes de palier (L3', L3'') fixes sont disposés symétriquement par rapport à un plan de symétrie fixé par le sens longitudinal (L) et le pivot (B).

Fig. 1

a)

b)

c)

d)

Fig. 2

Fig. 3

Fig. 4a

Fig. 4c

Fig. 4b

Fig. 5

Fig. 6

x-Position Hohlrad

y-Position Hohlrad

x-Position Betätigungselement 7

x-Position Betätigungselement 9

Umlaufwinkel des Hohlrads [°]

Fig. 7

Fig. 8

Fig. 9

Fig. 10

EP 3 498 147 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015215469 A1
- US 7297142 B2
- DE 102006027385 A1
- EP 1098429 A2